# EUROPÄISCHE PATENTSCHRIFT

(11) **EP 2 822 466 B1**
(45) Veröffentlichungstag und Bekanntmachung des Hinweises auf die Patenterteilung: **18.11.2015**
(21) Anmeldenummer: 13773655.9
(22) Anmeldetag: 25.09.2013
(51) Int. Cl.: A61B 6/02, A61B 6/00

(54) **VERFAHREN, VORRICHTUNG UND COMPUTERPROGRAMMPRODUKT ZUM AUFNEHMEN VON PROJEKTIONSBILDERN MIT OPTIMIERTER BEWEGUNGSBAHN**
METHOD, DEVICE AND COMPUTER PROGRAM PRODUCT FOR CAPTURING PROJECTION IMAGES WITH OPTIMISED MOVEMENT PATH
PROCÉDÉ, DISPOSITIF ET LOGICIEL D'ENREGISTREMENT D'IMAGES DE PROJECTION À TRAJECTOIRE DE DÉPLACEMENT OPTIMISÉE

(30) Priorität: 26.09.2012 DE 102012217490
(43) Veröffentlichungstag der Anmeldung: 14.01.2015
(73) Patentinhaber: FRAUNHOFER-GESELLSCHAFT zur Förderung der angewandten Forschung e.V., 80636 Munich (DE); Charité - Universitätsmedizin Berlin, 10117 Berlin (DE)
(72) Erfinder: STOPP, Fabian, 15366 Neuenhagen (DE); KÄSEBERG, Marc, 16359 Biesenthal (DE); ENGEL, Sebastian, 48147 Münster (DE); KEEVE, Erwin, 14469 Potsdam (DE); FEHLHABER, Felix, 10407 Berlin (DE); UHLMANN, Eckart, 25368 Kiebitzreihe (DE)
(74) Vertreter: Pfenning, Meinig & Partner GbR
(86) Internationale Anmeldenummer: PCT/EP2013/069928
(87) Internationale Veröffentlichungsnummer: WO 2014/048965

(56) Entgegenhaltungen:
- WO-A2-2007/130433
- US-A1- 2004 066 884
- US-A1- 2012 224 664
- US-B2- 7 342 992

## Beschreibung

Die vorliegende Erfindung betrifft ein Verfahren und eine Vorrichtung zum Aufnehmen von Projektionsbildern mit optimierter Bewegungsbahn. Außerdem betrifft die vorliegende Erfindung ein Computerprogrammprodukt, das eine Befehlsfolge enthält, die eine Vorrichtung zum Aufnehmen von Projektionsbildern ansteuert.

Zur dreidimensionalen Darstellung einzelner Objekte, wie beispielsweise einzelner Körperregionen, werden unterschiedliche Abbildungsmethoden verwendet. Eine mögliche Methode ist eine Kegelstrahlvolumentomographie, bei der einzelne zweidimensionale Röntgenprojektionen der abzubildenden Körperregion aus unterschiedlichen Richtungen aufgenommen werden und nachfolgend eine Berechnung einer dreidimensionalen Darstellung erfolgt. Zur Bildaufnahme bewegen sich eine Röntgenquelle und ein Röntgendetektor um das abzubildende Objekt. Eine hierbei verwendete Bildaufnahmebahn von der Röntgenquelle und dem Röntgendetektor um das abzubildende Objekt, beispielsweise einen Patienten, bestimmt wesentlich die erreichbare dreidimensionale Bildqualität der Kegelvolumenstrahltomographie.

Für eine exakte Rekonstruktion eines Volumenbereichs wurde von Heang K. Tuy (H. Tuy, "An inversion formula for cone-beam reconstruction", SIAM Journal on Applied Mathematics vol. 43, no. 3, pp. 546-552, 1983.), eine so genannte Vollständigkeitsbedingung formuliert. Diese besagt, dass ein Volumen exakt rekonstruierbar ist, wenn alle Ebenen, die durch das Volumen verlaufen, auch mindestens einmal die Bildaufnahme- bzw. Bewegungsbahn der Röntgenquelle schneiden. Eine derartige Bewegungsbahn ist allerdings schwierig umzusetzen, vor allem bei einer intraoperativen Anwendung der Kegelstrahlvolumentomographie im Operationssaal. In diesem Fall ist neben einer hohen dreidimensionalen Bildqualität eine Gewährleistung von Sterilität und eines freien Zugangs zum Patienten erforderlich, d. h. der Patient als das abzubildende Objekt sollte nicht durch eine zur Abbildung verwendete Vorrichtung oder die Bildaufnahmebahn vollständig umschlossen werden.

Aus dem Stand der Technik bekannte Geräte verwenden eine kreisförmige Bewegung des Röntgendetektors und der Röntgenquelle um den Patienten mit einer zusätzlichen linearen Fortbewegung (sodass sich z. B. eine Spiralbahn ergibt) oder einer zusätzlichen Neigung. Eine derartige Vorrichtung ist z. B. aus der Patentschrift US 6504892 B1 bekannt. Eine weitere Möglichkeit ist es, eine einfache Kreisbahn um den Patienten vorzusehen, bei der der Patient zwar nicht vollständig umschlossen wird, aber eine Rekonstruktion des gescannten Volumens ausschließlich in einer Zentralebene der Kreisbahn vollständig ist. Außerhalb dieser Zentralebene kann das Volumen nicht exakt rekonstruiert werden. Weitere Lösungsmöglichkeiten umfassen sattelförmige Bewegungsbahnen, die auch als Wobbeltrajektorien bezeichnet werden, oder auch eine Verwendung mehrerer einzelner Kreisbahnen der Röntgenquelle um die Zielregion, z. B. zwei parallel zueinander verlaufende Kreisbahnen.

Der vorliegenden Erfindung liegt damit die Aufgabe zugrunde, ein Verfahren und eine Vorrichtung vorzuschlagen, mit denen die genannten Nachteile vermieden werden, mit denen also eine hohe dreidimensionale Bildqualität bei freiem Zugang zu dem abzubildenden Objekt erreicht wird.

Diese Aufgabe wird erfindungsgemäß gelöst durch ein Verfahren nach Anspruch 1, eine Vorrichtung nach Anspruch 9 sowie ein Computerprogrammprodukt nach Anspruch 10 zum Steuern einer Vorrichtung bzw. zum Durchführen eines Verfahrens. Vorteilhafte Weiterbildungen werden in den Unteransprüchen beschrieben.

Ein erfindungsgemäßes Verfahren zum Aufnehmen von Projektionsbildern eines abzubildenden Objekts bedient sich einer Abbildungsvorrichtung, die eine Strahlenquelle, einen Strahlendetektor und eine Steuereinheit aufweist. Die Strahlenquelle wird durch die Steuereinheit auf einer Bahn in mehrere Positionen verfahren, in denen jeweils eine von der Strahlenquelle kegelstrahlförmig um einen Zentralstrahl ausgehende Strahlung ausgesandt wird, wobei mit dem Verfahren der Strahlenquelle der Strahlendetektor ebenfalls durch die Steuereinheit in mehrere Positionen verfahren wird, in denen jeweils die von der Strahlenquelle ausgehende und das abzubildende Objekt durchdringende Strahlung zum Aufnehmen eines Projektionsbilds aufgefangen wird. Die Strahlenquelle und bzw. oder der Strahlendetektor werden jeweils auf einer durch ein Polynom n-ten Grades beschriebenen und von der Steuereinheit durch eine Optimierung einer vorgegebenen Bahn berechneten Bahn, die einen entlang des Zentralstrahls bestimmten, konstanten Abstand zwischen der Strahlenquelle und dem Strahlendetektor und bzw. oder einen entlang des Zentralstrahls bestimmten, konstanten Abstand zwischen der Strahlenquelle und dem abzubildenden Objekt aufweist, um das abzubildende Objekt verfahren. Hierbei ist das Polynom jeweils derart gewählt, dass ein Sicherheitsabstand zu dem abzubildenden Objekt eingehalten wird und gleichzeitig ein Abstand zwischen dem Strahlendetektor und dem abzubildenden Objekt minimiert wird. Unter einem Polynom "n-ten" Grades soll dabei ein beliebiges geradzahliges Polynom verstanden werden, "n" also eine natürliche Zahl sein.

Durch die polynomielle Bewegungsbahn des Strahlendetektors ist dieser während einer Bildaufnahme näher am abzubildenden Objekt als bei bislang verwendeten Verfahren, wodurch auch das auf den Projektionsbildern abbildbare Volumen vergrößert wird. Die polynomielle Bahn wird hierbei durch die Steuereinheit berechnet. Durch dieses Verfahren wird die Vollständigkeitsbedingung von Tuy zumindest für ein Zielvolumen im Zentrum der Bahn erfüllt und somit kann in einem weiteren Schritt eine qualitativ hochwertige, exakte dreidimensionale Rekonstruktion des abzubildenden Objekts erfolgen. Die Verwendung der polynomiellen Bewegungsbahn erlaubt eine Optimierung der Bewegungsbahn in einem weiten Bereich unter Berücksichtigung von Umgebungsbedingungen. Durch den konstanten Abstand zwischen der Strahlenquelle und dem abzubildenden Objekt bzw. zwischen der Strahlenquelle und dem Strahlendetektor wird eine Berechnung der Bewegungsbahn vereinfacht und gleichzeitig das Einhalten des Sicherheitsabstands gewährleistet. Im Vergleich zu bekannten Verfahren aus dem Stand der Technik ist ein Volumenbereich, der die Vollständigkeitsbedingung erfüllt, bei dem hier vorgestellten Verfahren größer, sodass auch ein größeres Volumen exakt rekonstruiert werden kann. Der Sicherheitsabstand, der zwischen dem Strahlendetektor und bzw. oder der Strahlenquelle und dem abzubildenden Objekt eingehalten werden soll, kann für die gesamte Bewegung konstant sein, alternativ aber auch für jeden Punkt der Bewegung einen vorab bestimmten Wert aufweisen, wobei dieser Wert für verschiedene Punkte verschieden groß ist. Somit wird stets ein bestimmter Abstand eingehalten und Kollisionen werden vermieden. Unter einer Minimierung des Abstands zwischen dem Strahlendetektor und dem abzubildenden Objekt soll verstanden werden, dass der Abstand möglichst nahe an dem Sicherheitsabstand liegt, also eine möglichst geringe Distanz zwischen dem Strahlendetektor und dem Objekt vorliegt.

Typischerweise erfolgt das Verfahren der Strahlenquelle und das Verfahren des Strahlendetektors gleichzeitig, es kann jedoch auch vorgesehen sein, die genannten Geräte zeitlich versetzt zu bewegen. Das gleichzeitige Verfahren erlaubt eine schnelle Durchführung des Verfahrens, während ein nacheinander erfolgendes Verfahren der Geräte die Möglichkeit gibt, noch Modifikationen am abzubildenden Objekt durchzuführen, bei denen eines der Geräte im anvisierten Endzustand einen Zugang erschweren würde.

Das abzubildende Objekt kann auf einer Auflagefläche zum verbesserten und sicheren Halten abgelegt sein, wobei in diesem Fall ein Sicherheitsabstand zu der Auflagefläche zusätzlich oder alternativ zu dem Sicherheitsabstand zu dem abzubildenden Objekt durch die polynomielle Bahn eingehalten wird. Die Auflagefläche ist typischerweise ein Tisch, insbesondere ein Tisch in einem Operationssaal, kann jedoch auch weitere Ausstattung des Operationssaals umfassen.

Es kann vorgesehen sein, dass die polynomielle Bewegungsbahn eine Bewegungsbahn bezüglich eines Zentrums einer Bildebene des Strahlendetektors beschreibt. Eine Bedingung für die Einstellung des Sicherheitsabstands kann dabei sein, dass der Strahlendetektor und bzw. oder die Strahlenquelle, typischerweise eine Oberfläche eines Gehäuses der genannten Geräte, an jeder Position der Bewegungsbahn der Strahlenquelle mindestens 80 mm, vorzugsweise mindestens 100 mm, besonders vorzugsweise mindestens 120 mm von dem abzubildenden Objekt und bzw. oder der Auflagefläche entfernt ist. Natürlich kann der Sicherheitsabstand auch kleiner als 80 mm gewählt werden.

Eine weitere Bedingung kann darin bestehen, dass ein maximaler Abstand zwischen der Strahlenquelle und bzw. oder dem Strahlendetektor und dem abzubildenden Objekt und bzw. oder ein möglicher Arbeitsbereich einer Kinematik der Strahlenquelle und bzw. oder des Strahlendetektors berücksichtigt werden, d. h. dass dieser maximale Abstand beim Verfahren der Strahlenquelle bzw. des Strahlendetektors nicht überschritten werden darf.

Die Abstände können jeweils von einem Mittelpunkt des jeweiligen Objekts zu einem weiteren Mittelpunkt eines anderen Objekts, einer Oberfläche eines Objekts zu einer weiteren Oberfläche eines weiteren Objekts oder dem Mittelpunkt des Objekts zu der weiteren Oberfläche des weiteren Objekts gemessen werden.

Das Polynom kann jeweils durch mindestens zwei Stützstellen definiert sein, die typischerweise extern vorgegeben sind oder vorgegeben werden, und vorzugsweise durch eine Optimierung unter Berücksichtigung der Stützstellen und bzw. oder durch eine Optimierung einer vorgegebenen kreisförmigen Bahn bestimmt werden. Bei der Optimierung mehrerer Bahnen kann jedes der Polynome oder auch nur ein einzelnes in der zuvor beschriebenen Weise durch mindestens zwei Stützstellen optimiert werden. Die Optimierung kann dabei direkt durch die bereits beschriebenen Bedingungen und bzw. oder über die vorgegebenen Stützstellen bei anschließender Überprüfung eines Polynomverlaufs geschehen.

Als Polynome können Polynome mit ausschließlich geraden Potenzen oder Polynome mit ausschließlich ungeraden Potenzen verwendet werden. Natürlich sind auch Polynome mit sowohl geraden als auch ungeraden Potenzen möglich. Je nach Anwendungszweck wird somit eine Optimierung der polynomiellen Bahn erleichtert.

Ein Ursprung eines Bezugskoordinatensystems, in dem die Bewegungsbahn festgelegt und optimiert wird, kann sich in dem Mittelpunkt des abzubildenden Objekts oder mittig auf der Auflagefläche befinden. Hierdurch wird ein einfach zu bestimmender fixer Punkt zur vereinfachten Berechnung festgelegt.

Sofern bei der Bewegungsbahn der Strahlenquelle von der Kreisbahn ausgegangen wird, kann diese Kreisbahn durch ein Polynom mittels der bereits beschriebenen Bedingungen optimiert werden. Statt einer kreisförmigen Bahn kann auch eine Bahn aus abschnittsweise linearen Teilstücken über das Polynom n-ten Grades angenähert und bzw. oder optimiert werden und somit die berechnete Bahn bilden, auf der die Strahlenquelle bzw. der Strahlendetektor verfahren werden.

Typischerweise sind die Strahlenquelle und der Strahlendetektor stets einander gegenüberliegend geführt und der Zentralstrahl trifft orthogonal auf den Strahlendetektor. Hierdurch wird eine dreidimensionale Rekonstruktion erleichtert. Es kann jedoch auch vorgesehen sein, dass der Zentralstrahl nicht orthogonal, also unter einem von 90° verschiedenen Winkel auf den Strahlendetektor auftrifft. Die Bewegung der Strahlenquelle erfolgt vorzugsweise separiert von der Bewegung des Strahlendetektors, um eine möglichst freie Einstellung der Bewegungen zu gewährleisten.

Es kann vorgesehen sein, dass eine maximale Höhe der Bahn des Strahlendetektors durch einen ausgehend von dem Mittelpunkt des abzubildenden Objekts bestimmten maximalen Öffnungswinkel der Bahn der Strahlenquelle bestimmt wird. Die maximale Höhe kann zum Festlegen von einem Startpunkt, einem Endpunkt und bzw. oder einem Umkehrpunkt der Bahn der Strahlenquelle und bzw. oder der Bahn des Strahlendetektors verwendet werden. Somit werden durch bauartbedingte Vorgaben einzelne Punkte der Bewegungsbahn zumindest bis zu einem gewissen Grad definiert. Vorzugsweise liegen der Startpunkt und der Endpunkt der Bahn des Strahlendetektors dem Startpunkt und dem Endpunkt der Bahn der Strahlenquelle entlang des durch den Mittelpunkt des abzubildenden Objekts verlaufenden Zentralstrahls gegenüber. Der Öffnungswinkel soll hierbei den Winkel beschreiben, der zwischen zwei in einer Ebene liegenden Strahlen verläuft, die ausgehend von dem Mittelpunkt des abzubildenden Objekts als gemeinsamen Anfangspunkt zu dem Startpunkt und dem Endpunkt der Bahn des Strahlendetektors verlaufen. Typischerweise liegt der Umkehrpunkt der Bahn des Strahlendetektors dem Umkehrpunkt der Bahn der Strahlenquelle gegenüber. Hierdurch wird eine geometrisch besonders einfache Form einer Zuordnung gewählt.

Die maximale Höhe kann ausgehend von dem Mittelpunkt des abzubildenden Objekts als einem Scanzentrum in Bezug auf die Auflagefläche bzw. in Bezug auf das Bezugskoordinatensystem durch zwei Verfahren bestimmt werden.

In einem ersten Verfahren wird anhand eines möglichen oder erforderlichen Arbeitsbereichs der Kinematik des Strahlendetektors die maximale Höhe des Detektors als Endstellungen festgelegt. Durch diese Höhe, vorzugsweise maximal 250 mm, besonders vorzugsweise maximal 150 mm über dem Mittelpunkt des abzubildenden Objekts oder einer Kante der Auflage, wird nachfolgend unter Berücksichtigung der Lage des Scanzentrums der maximale Öffnungswinkel der Bahn der Strahlenquelle festgelegt. Je niedriger die Höhe des Scanzentrums und je höher die Endstellungen des Strahlendetektors, desto größer ist der maximale Öffnungswinkel.

In einem zweiten Verfahren wird ein maximaler Öffnungswinkel definiert, der umgesetzt werden soll. Anschließend werden Schnittpunkte des Zentralstrahls der Strahlenquelle ausgehend von dem Startpunkt und dem Endpunkt der Bahn der Strahlenquelle bestimmt. Diese zwei Schnittpunkte definieren erforderliche Maximalstellungen des Mittelpunktes des Detektors und somit die erforderliche Höhe der Bahn des Detektors. Durch die genannten Verfahren lassen sich Einschränkungen definieren, die die Optimierung vereinfachen.

Die Bewegung der Strahlenquelle und bzw. oder des Strahlendetektors kann offen sein, d. h. auf einer offenen Bahn erfolgen. Unter einer offenen Bahn ist hierbei eine Bahn zu verstehen, bei der der Anfangspunkt und der Endpunkt der Bahn voneinander verschieden sind und bzw. oder die Bahn das abzubildende Objekt nicht komplett umschließt. Hierdurch ist ein Systemaufbau zur Umsetzung des Bildaufnahmeverfahrens möglich, der das abzubildende Objekt nicht vollständig umschließt. Somit ist ein Zugang zu dem abzubildenden Objekt stets gewährleistet. Alternativ kann die Bewegung der Strahlenquelle und bzw. oder des Strahlendetektors auch auf einer geschlossenen Bahn verlaufen, bei der Anfangspunkt und Endpunkt übereinstimmen.

Alternativ oder zusätzlich kann die Bewegung der Strahlenquelle und bzw. oder des Strahlendetektors mindestens einen Umkehrpunkt aufweisen, in dem ein Richtungswechsel stattfindet. Hierdurch wird eine erhöhte Qualität einer dreidimensionalen Rekonstruktion erreicht. Vorzugsweise ist hierbei ein erster Bahnabschnitt bis zu dem Umkehrpunkt räumlich verschieden von einem zweiten Bahnabschnitt ab dem Umkehrpunkt, sodass mehrere verschiedene Positionen überdeckt werden.

Es kann auch vorgesehen sein, dass der Strahlendetektor und bzw. oder die Strahlenquelle entlang von und bzw. oder um mindestens zwei, vorzugsweise senkrecht aufeinander stehender Achsen bewegt wird. Durch diese einfache geometrische Relation der Achsen und somit der Bewegung wird sowohl eine Ansteuerung als auch eine später eventuell erfolgende Auswertung erheblich vereinfacht. Die Bewegungen um die beiden Achsen können auch von unterschiedlichem Typ sein, also beispielsweise eine Linearbewegung und eine Rotationsbewegung umfassen.

Eine Neigung der Strahlenquelle wird typischerweise derart eingestellt, dass eine bestrahlte Fläche des Strahlendetektors bei einem festen Winkel des Kegelstrahls maximiert ist. Durch den festen Winkel wird eine räumliche Ausdehnung des Kegeistrahls in Abhängigkeit von der Strahlenquelle definiert, sodass durch die Neigung der Strahlenquelle eine komplette Fläche des Strahlendetektors zur maximalen Informationsgenerierung bestrahlt wird.

Die Steuereinheit kann zumindest eine der Bewegungen der Strahlenquelle und bzw. oder des Strahlendetektors automatisiert durchführen, sodass ohne weiteres menschliches Zutun die Projektionsbilder erhalten werden. Vorzugsweise werden jedoch beide Bewegungen automatisiert von der Steuereinheit durchgeführt. Alternativ oder zusätzlich kann eine Auswerteeinheit die dreidimensionale Rekonstruktion des abzubildenden Objekts aus den Projektionsbildern berechnen. Typischerweise sind die Steuereinheit und die Auswerteeinheit in einem Gerät kombiniert, vorzugsweise in einem Computer.

Typischerweise ist die Strahlenquelle eine Röntgenquelle und eine ausgesandte Strahlung Röntgenstrahlung, sowie der Strahlendetektor ein Röntgendetektor, vorzugsweise ein Röntgenflachdetektor.

Eine Vorrichtung zum Aufnehmen von Projektionsbildern umfasst einen Strahlendetektor, eine Strahlenquelle und eine Steuereinheit, wobei die Steuereinheit eingerichtet ist, den Strahlendetektor und die Strahlenquelle jeweils in mehrere Positionen zu verfahren und in jeder der Positionen ein Projektionsbild aufzunehmen. Die Strahlenquelle ist dazu eingerichtet, eine Strahlung kegelstrahlförmig um einen Zentralstrahl auszusenden und der Strahlendetektor ist eingerichtet, die ausgesandte und das abzubildende Objekt durchdringende Strahlung aufzufangen. Die Steuereinheit ist dazu ausgebildet, die Strahlenquelle und den Strahlendetektor jeweils auf einer durch ein Polynom n-ten Grades beschriebenen und von der Steuereinheit durch eine Optimierung einer vorgegebenen Bahn berechneten Bahn um das abzubildende Objekt zu verfahren, wobei die berechnete Bahn einen entlang des Zentralstrahls bestimmten, konstanten Abstand zwischen der Strahlenquelle und dem Strahlendetektor und bzw. oder einen entlang des Zentralstrahls bestimmten, konstanten Abstand zwischen der Strahlenquelle und dem abzubildenden Objekt aufweist, wobei das Polynom jeweils derart gewählt ist, einen Sicherheitsabstand zu dem abzubildenden Objekt einzuhalten und gleichzeitig einen Abstand zwischen dem Strahlendetektor und dem abzubildenden Objekt zu minimieren. Die Minimierung erfolgt typischerweise auf den Sicherheitsabstand hin, so dass der Strahlendetektor möglichst nahe an dem abzubildenden Objekt liegt. Somit dient die Vorrichtung zur einfachen Aufnahme hochwertiger Projektionsbilder, aus denen auch qualitativ hochwertige dreidimensionale Rekonstruktionen berechnet werden können.

Typischerweise ist die Vorrichtung zum Durchführen des beschriebenen Verfahrens geeignet.

Vorzugsweise umfasst die Vorrichtung die Auswerteeinheit mit den bereits beschriebenen Eigenschaften zum Berechnen der dreidimensionalen Rekonstruktionen.

Ein Computerprogrammprodukt enthält eine Befehlsfolge, die eine Vorrichtung zum Aufnehmen von Projektionsbildern, umfassend einen Strahlendetektor, eine Strahlenquelle und eine Steuereinheit, wobei die Steuereinheit eingerichtet ist, den Strahlendetektor und die Strahlenquelle jeweils in mehrere Positionen zu verfahren und in jeder der Positionen ein Projektionsbild aufzunehmen und die Strahlenquelle eingerichtet ist, eine Strahlung kegelstrahlförmig um einen Zentralstrahl auszusenden, und der Strahlendetektor eingerichtet ist, die ausgesandte Strahlung zu detektieren, derart steuert, dass die Strahlenquelle und der Strahlendetektor jeweils auf einer durch ein Polynom n-ten Grades beschriebenen und von der Steuereinheit durch eine Optimierung einer vorgegebenen Bahn berechneten Bahn um das abzubildende Objekt verfahren werden, wobei die berechnete Bahn einen entlang des Zentralstrahls bestimmten, konstanten Abstand zwischen der Strahlenquelle und dem Strahlendetektor und/oder einen entlang des Zentralstrahls bestimmten, konstanten Abstand zwischen der Strahlenquelle und dem abzubildenden Objekt aufweist. Hierbei ist das Polynom derart gewählt, einen Sicherheitsabstand zu dem abzubildenden Objekt einzuhalten und gleichzeitig einen Abstand zwischen dem Strahlendetektor und dem abzubildenden Objekt zu minimieren. Die Minimierung erfolgt typischerweise auf den Sicherheitsabstand hin, so dass der Strahlendetektor möglichst nahe an dem abzubildenden Objekt liegt und der Abstand zwischen dem Strahlendetektor und dem abzubildenden Objekt möglichst nahe an dem Sicherheitsabstand liegt.

Das Computerprogrammprodukt ist auch zur Durchführung des beschriebenen Verfahrens geeignet. Typischerweise wird das Computerprogrammprodukt zum Steuern der bereits beschriebenen Vorrichtung eingesetzt. Das Durchführen des Verfahrens und bzw. oder das Ansteuern der Vorrichtung durch das Computerprogrammprodukt erfolgt typischerweise, wenn das Computerprogrammprodukt auf einer Recheneinheit abläuft.

Vorzugsweise kann das Computerprogrammprodukt direkt in einen internen Speicher der Recheneinheit geladen werden oder ist in diesem bereits gespeichert und umfasst typischerweise Teile eines Programmcodes zum Durchführen des beschriebenen Verfahrens oder zum Ansteuern der beschriebenen Vorrichtung, wenn das Computerprogrammprodukt auf der Recheneinheit abläuft bzw. ausgeführt wird. Das Computerprogrammprodukt kann auf einem maschinenlesbaren Träger, vorzugsweise einem digitalen Speichermedium gespeichert sein. Das Computerprogrammprodukt kann auch ein Computerprogramm umfassen, das Softwaremittel zur Durchführung des beschriebenen Verfahrens und bzw. oder zum Ansteuern der beschriebenen Vorrichtung aufweist, wenn das Computerprogramm in einem Automatisierungssystem oder auf der Recheneinheit ausgeführt wird.

Das beschriebene Verfahren, die beschriebene Vorrichtung und bzw. oder das beschriebene Computerprogrammprodukt finden vorzugsweise Verwendung bei medizinischen Anwendungen, insbesondere bei intraoperationellen Anwendungen, vorzugsweise bei einer Kegelstrahlvolumentomographie. Medizinische Anwendungsgebiete sind insbesondere Unfallchirurgie, Mundchirurgie, Kieferchirurgie, Gesichtschirurgie, Orthopädie, Neurologie, Urologie, Kardiologie oder Notfallscans bei einem Polytrauma.

Ausführungsbeispiele der Erfindung sind in den Zeichnungen dargestellt und werden nachfolgend anhand der Figuren 1-12 erläutert.

Es zeigen:
- Fig. 1: eine perspektivische Ansicht einer Vorrichtung zum Aufnehmen von Röntgenbüdern;
- Fig. 2: eine Schnittdarstellung eines OP-Tischs mit einer Bewegungsbahn einer Röntgenquelle und einer Bewegungsbahn eines Röntgendetektors;
- Fig. 3: eine Ansicht der in Fig. 2 gezeigten Bewegungsbahnen von Röntgenquelle und Röntgendetektor in einem Bezugskoordinatensystem;
- Fig. 4: eine Fig. 3 entsprechende Darstellung der Bewegungsbahnen der Röntgenquelle und des Röntgendetektors bei konstantem Abstand zwischen der Röntgenquelle und dem Röntgendetektor;
- Fig. 5: eine Fig. 2 entsprechende Darstellung der in Fig. 4 wiedergegebenen Bewegungsbahnen;
- Fig. 6: eine perspektivische Darstellung des OP-Tischs mit den Bewegungsbahnen der Röntgenquelle und des Röntgendetektors;
- Fig. 7: eine Fig. 1 entsprechende Darstellung der Vorrichtung mit den Bewegungsbahnen;
- Fig. 8: eine Fig. 2 entsprechende Darstellung von weiteren Ausführungsformen der Bewegungsbahnen mit Umkehrpunkten;
- Fig. 9: eine seitliche Ansicht des OP-Tischs mit den in Fig. 8 dargestellten Bewegungsbahnen;
- Fig. 10: eine seitliche Ansicht der Vorrichtung mit den in den Fign.8 und 9 gezeigten Bewegungsbahnen;
- Fig. 11: eine Schnittdarstellung des OP-Tischs mit eingezeichneten Sicherheitsabständen und
- Fig. 12: eine Fig. 2 entsprechende Ansicht des OP-Tischs mit einer weiteren Ausführungsform einer ursprünglichen Bewegungsbahn des Röntgendetektors.

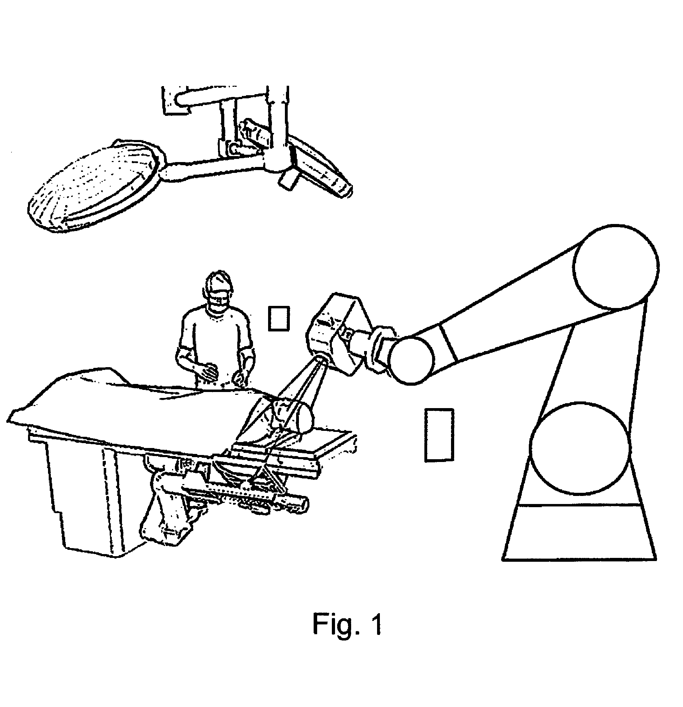

Fig. 1 zeigt in einer perspektivischen Ansicht eine Vorrichtung zum Aufnehmen von Röntgenbildern, die zunächst als zweidimensionale Projektionsbilder aufgenommen werden und anschließend zu dreidimensionalen Bildern weiterverarbeitet werden. Die Vorrichtung umfasst hierzu einen Röntgendetektor 1 als Strahlendetektor, eine Röntgenquelle 2 als Strahlenquelle, eine Steuereinheit 3, die zusammen mit einer Auswerteeinheit 4 einen Roboterarm 5 steuert und einen Tisch in einem Operationssaal, also einen OP-Tisch 6 als Auflagefläche für das abzubildende Objekt, in dem in Fig.1 dargestellten Ausführungsbeispiel einen Patienten 7. Statt der Röntgenquelle 2 und dem Röntgendetektor 1 können natürlich auch andere Strahlenquellen und -detektoren Verwendung finden.

Der Röntgendetektor 1 ist ein digitaler Röntgenflachdetektor und ist unterhalb des OP-Tischs 6 auf einer Führung verschiebbar gelagert, kann aber natürlich auch ortsfest mit dem OP-Tisch 6 verbunden sein. Zum Verfahren ist der Röntgendetektor 1 auf einer fünfachsigen Kinematik geführt, die den Röntgendetektor 1 automatisiert bewegen kann. Im dargestellten Ausführungsbeispiel entspricht der Röntgendetektor 1 und seine Führung den entsprechenden Komponenten der deutschen Patentanmeldung 10 2012 005 899.3. Insbesondere kann der Röntgendetektor 1 auch so verfahren werden, dass er sich neben dem abzubildenden Objekt befindet.

Die Röntgenquelle 2 sendet Röntgenstrahlung kegelstrahlförmig um einen Zentralstrahl aus. Diese Röntgenstrahlung durchdringt einen Kopf des Patienten 7 bzw. einen anderen Teil seines Körpers und wird von dem Röntgendetektor 1 detektiert. Eine so erhaltene zweidimensionale Projektionsaufnahme wird über ein Kabel 8 oder auch kabellos an die Auswerteeinheit 4, im dargestellten Beispiel einen Personalcomputer, übergeben zur weiteren Auswertung.

Die Röntgenquelle 2 ist am Ende des Roboterarms 5 befestigt, wodurch die Röntgenquelle 2 automatisiert bewegt werden kann. Der Roboterarm 5 ist ein Arm eines Industrieroboters und beweglich auf einem Boden des Operationssaals gelagert. Der Roboterarm 5, die Röntgenquelle 2, der OP-Tisch 6 und der Röntgendetektor 1 entsprechen in dem in Fig. 1 gezeigten Ausführungsbeispiel den entsprechenden Komponenten der Druckschrift DE 10 2010 018 627 A1. Insbesondere ist der Röntgenstrahler 2 in mindestens vier Freiheitsgraden bewegbar.

Der Roboterarm 5 wird über die Steuereinheit 3 gesteuert, die Steuersignale entweder über ein Kabel 9 oder kabellos an den Roboterarm 5 übermittelt. Die Steuereinheit 3 enthält hierfür ein Computerprogrammprodukt mit einer Befehlsfolge, das die beschriebene Vorrichtung zum Durchführen des Verfahrens ansteuert. Das Computerprogrammprodukt ist auf einem maschinenlesbaren Träger wie z. B. einer Festplatte, einer CD oder einem USB-Stick gespeichert und umfasst Teile eines Programmcodes, der die Vorrichtung wie beschrieben ansteuert und das beschriebene Verfahren durchführt, wenn das Computerprogrammprodukt auf der Steuereinheit 3 abläuft. Die Steuereinheit 3 entspricht dem Personalcomputer, der auch die Auswerteeinheit 4 bildet, kann jedoch auch als separate Vorrichtung ausgeführt sein.

Ein Benutzer 10 der Vorrichtung, beispielsweise ein Arzt oder dessen Assistent im Operationssaal, kann somit sowohl zweidimensionale Projektionsbilder, die in verschiedenen Positionen der Röntgenquelle 2 und des Röntgendetektors 1 gemacht wurden, als auch durch die Auswerteeinheit 4 erstellte dreidimensionale Abbildungen eines aus mehreren Positionen abgebildeten Zielvolumens auf einem Bildschirm 11, der mit der Auswerteeinheit 4 durch ein Kabel 38 verbunden ist, intraoperationell, also während eines Eingriffs am Patienten 7 anschauen. Generierte Volumendaten des Zielvolumens des Patienten 7 können natürlich nicht nur während des chirurgischen Eingriffs, sondern auch bereits davor oder danach durch die in Fig. 1 dargestellte Vorrichtung und das im Folgenden beschriebene Verfahren erhalten werden. Medizinische Anwendungsgebiete sind hierbei typischerweise Unfallchirurgie, Mundchirurgie, Kieferchirurgie, Gesichtschirurgie, Orthopädie, Neurologie, Urologie, Kardiologie oder Notfallscans bei einem Polytrauma.

Die Steuereinheit 3 ist dazu ausgebildet, die Röntgenquelle 2 und den Röntgendetektor 1 jeweils auf einer durch ein Polynom n-ten Grades beschriebenen Bahn um den Patienten 7 zu verfahren. Hierzu berechnet die Steuereinheit 3 auch diese genannten Bahnen, üblicherweise mittels einer Optimierung einer vorgegebenen Bahn. Dieses Polynom n-ten Grades kann in einem Verfahrensschritt bestimmt oder festgelegt werden.

Mit der in Fig. 1 gezeigten Vorrichtung kann somit ein Verfahren durchgeführt werden, bei dem die Projektionsbilder des Patienten 7 aus mehreren Positionen aufgenommen werden. Die Steuereinheit 3 verfährt hierzu gleichzeitig den Röntgendetektor 1 und die Röntgenquelle 2 in eine der Positionen, die Röntgenquelle 2 sendet in Richtung des Patienten 7 Röntgenstrahlung kegelstrahlförmig um einen Zentralstrahl aus, die den Patienten 7 zumindest teilweise durchdringt und teilweise absorbiert wird, wobei der Röntgendetektor 1 nicht-absorbierte Anteile detektiert. Anschließend werden durch die Steuereinheit 3 die Röntgenquelle 2 und der Röntgendetektor 1 in eine weitere Position verfahren und wiederum eine der Projektionsaufnahmen aufgenommen. Die Röntgenquelle 2 und der Röntgendetektor 1 werden jeweils auf einer polynomiellen Bewegungsbahn geführt, die jeweils durch eine nachfolgend noch genauer spezifizierte Optimierung einer vorgegebenen Bahn erfolgt, d. h. sowohl die Bewegungsbahn der Röntgenquelle 2 als auch die Bewegungsbahn des Röntgendetektors 1 werden für sich genommen optimiert. In weiteren Ausführungsbeispielen kann natürlich auch nur eine der beiden Bahnen polynomiell optimiert werden. In weiteren Ausführungsformen können der Röntgendetektor 1 und die Röntgenquelle 2 auch nacheinander verfahren werden.

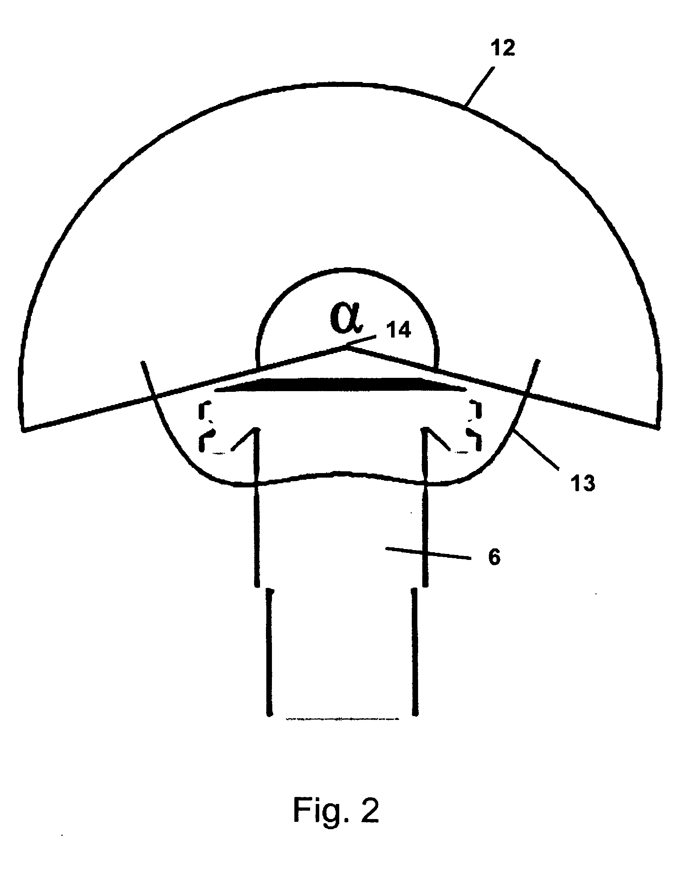

Fig. 2 zeigt in einer Schnittdarstellung des OP-Tischs 6 eine weitestgehend oberhalb des OP-Tischs 6 verlaufende Bahn 12 der Röntgenquelle 2 und eine weitestgehend unterhalb des OP-Tischs 6 verlaufende Bahn 13 des Röntgendetektors 1. Wiederkehrende Merkmale sind in dieser wie auch in den folgenden Figuren jeweils mit gleichen Bezugszeichen versehen. Die Bahn 12 der Röntgenquelle 2 beschreibt einen Kreisbogen mit einem Öffnungswinkel α von 196°, der sich aus einem Kreisbogen von 180° plus einem Kegelstrahlöffnungswinkel γ von 16° ergibt. Ein Mittelpunkt des Kreisbogens liegt in einem Mittelpunkt 14 des Zielvolumens und dient als Ursprung eines Bezugskoordinatensystems, sodass ein Abstand zwischen diesem Mittelpunkt 14 und der Röntgenquelle 2 während des Abfahrens der Bahn konstant bleibt. Die Bahn 12 der Röntgenquelle 2 verläuft in einer Ebene. Die Bahn 13 des Röntgendetektors 1 verläuft in einer geschwungenen Linie unterhalb des OP-Tischs 6, weist jedoch zwei Seitenstücke auf, die neben dem OP-Tisch 6, und sofern der Patient 7 darauf aufliegt, auch neben diesem liegen. Die Bahn 13 des Röntgendetektors 1 ist ebenfalls planar in einer Ebene verlaufend, wobei die Ebene dieser Bahn identisch mit der Ebene der Bahn 12 der Röntgenquelle 2 ist. Statt der in Fig. 2 gezeigten Bahn 13 kann der Röntgendetektor 1 auch auf einer kreisbogenförmigen Bahn um den Patienten 7 geführt werden, wobei ein Abstand zwischen dem Röntgendetektor 1 und dem Mittelpunkt 14 des Zielvolumens konstant ist. Der Ursprung des Bezugskoordinatensystems kann jedoch in weiteren Ausführungsbeispielen auch mittig auf dem OP-Tisch 6 liegen.

Ausgehend von der planaren Aufnahmebahn 12 der Röntgenquelle 2, entlang derer die Röntgenquelle 2 in mehrere Positionen zum Aufnehmen der Projektionsbilder bewegt wird, kann in einem Optimierungsverfahren die Bewegungsbahn 13 des Röntgendetektors 1 abgeändert werden und von einer polynomiellen Funktion n-ten Grades beschrieben werden. Die polynomielle Bahn verläuft um den Patienten 7 mit dem Zielvolumen und auch um den OP-Tisch 6. Das Polynom ist derart definiert, dass zum einen ein Sicherheitsabstand zu dem OP-Tisch 6 oder anderen Geräten des Operationssaals bzw. Personen im Operationssaal gewährleistet wird und zum anderen eine möglichst geringe Distanz zu dem Objekt auf dem OP-Tisch 6, also dem Patienten 7, vorhanden ist. Dies erfolgt in gleicher Weise für den Röntgenstrahler 2. Die Distanz zwischen der Röntgenquelle 2 und dem Röntgendetektor 1 ist im Idealfall soweit minimiert, dass sie gerade dem einzuhaltenden Sicherheitsabstand zwischen den genannten Vorrichtungen und dem OP-Tisch oder dem Patienten 7 bzw. anderen Geräten oder Personen entspricht.

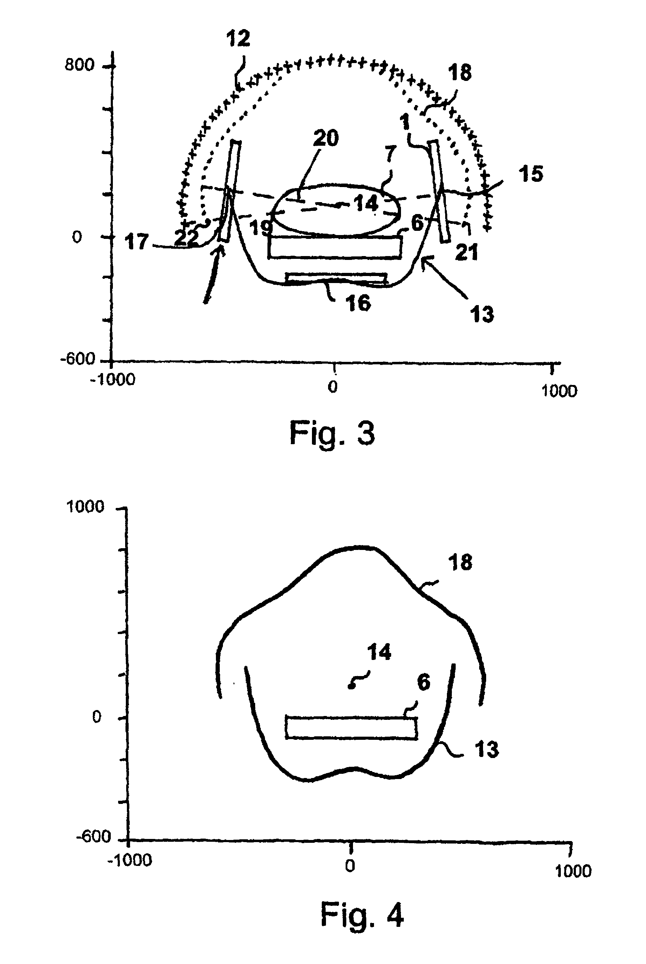

In Fig. 3 ist in dem Bezugskoordinatensystem die bereits angepasste Bahn 13 des Röntgendetektors 1 sowie die ursprüngliche Bahn 12 und eine angepasste polynomielle Bahn 18 der Röntgenquelle 2 durch einzelne der angefahrenen Positionen dargestellt. Der Patient 7 liegt auf dem OP-Tisch 6 auf. Der Mittelpunkt 14 des eingezeichneten Koordinatensystems fällt zusammen mit dem Mittelpunkt des Zielvolumens des Patienten 7 als dem abzubildenden Objekt. Wie in Fig. 1 dargestellt, kann das Zielvolumen der Kopf sein. Der Mittelpunkt des Zielvolumens kann dabei sowohl ein geometrischer Mittelpunkt als auch ein Masseschwerpunkt sein. Die Bahn 13 des Röntgendetektors 1 ist bereits durch eine von der Steuereinheit 3 ausgeführte Berechnung der Bahn 13 optimiert und startet mit der eingezeichneten Stellung 15 des Röntgendetektors 1 rechts des Patienten 7. Entlang der Bahn 13 wird der Röntgendetektor 1 über eine Mittelposition 16 unterhalb des Patienten 7 in eine Endposition 17 links neben dem Patienten 7 geführt. Die Bahn 13 verläuft symmetrisch zu der Mittelposition 16. Die ursprüngliche Bahn 12 der Röntgenquelle 2 vor der Optimierung ist durch Kreuze angezeigte Positionen in Fig. 3 dargestellt. Die nach der Optimierung erhaltene polynomielle Bahn 18 ist durch Punkte gekennzeichnet. In einem Mittelteil oberhalb des OP-Tischs 6 verlaufen die Bahnen 12 und 18 gleich, ansonsten liegen die Positionen der Bahn 18 näher an dem OP-Tisch 6 als die korrespondierenden Positionen der Bahn 12. Die Bestimmung der polynomiellen Funktionen kann über geeigneten Stützstellen geschehen, im vorliegenden Beispiel die Positionen der Bahn 18, die mit Positionen der ursprünglichen Bahn 12 zusammenfallen, und bzw. oder über eine direkte Optimierung der polynomiellen Bewegungsbahn, d. h. eine Optimierung ohne Vorgabe von Stützstellen, unter Berücksichtigung des vorgegebenen Sicherheitsabstands zu dem OP-Tisch 6 und dem Patienten 7. Diese Stützstellen werden im dargestellten Ausführungsbeispiel in einem separaten Verfahrensschritt vorgegeben und bei der Berechnung der Bahn berücksichtigt. Typischerweise halten sowohl der Röntgendetektor 1 als auch die Röntgenquelle 2 den Sicherheitsabstand zu dem abzubildenden Objekt ein, wobei der Sicherheitsabstand zwischen der Röntgenquelle und dem abzubildenden Objekt größer, kleiner oder gleich dem Sicherheitsabstand zwischen dem Röntgendetektor und dem abzubildenden Objekt sein kann.

Das zur Optimierung verwendete Polynom kann nur geradzahlige Potenzen, nur ungeradzahlige Potenzen oder beide Arten von Potenzen enthalten. Ein Beispiel für ein optimiertes Polynom 6. Grades als Ergebnispolynom der Optimierung für die Bahn 13 des Röntgendetektors 1 ist
y=9,805256*10⁻¹⁵*x⁶+1,150705*10⁻⁸*x⁴-1,10097*10⁻³*x²-200.

Hierbei wurden in dem in Figur 3 eingezeichneten Koordinatensystem die Stützstellen für die Bahn 13 des Röntgendetektors 1 (0, -200), (-200, -225), (200, -225), (-375,-100), (375, -100), (-475, 250) und (475, 250) verwendet. Der Ursprung des Bezugskoordinatensystems befindet sich dabei in einer Mitte einer Oberfläche des OP-Tischs 6. Der Sicherheitsabstand des Röntgendetektors 1 beträgt in dem in Fig. 3 gezeigten Ausführungsbeispiel 80 mm, kann aber je nach Situation auch größer oder kleiner sein.

Eine maximale Höhe der polynomiellen Bewegungsbahnen 13 und 18 der Röntgenquelle 2 und des Röntgendetektors 1 wird typischerweise durch den maximalen Öffnungswinkel α und die Lage des Zielvolumens bestimmt: Der Startpunkt 15 und der Endpunkt 17 der Bahn 13 des Röntgendetektors 1 ergeben sich als Schnittpunkte mit den durch den Mittelpunkt 14 verlaufenden Zentralstrahlen 19 und 20, die die Röntgenquelle 2 in einem Startpunkt 22 der Bahn 18 oder der Bahn 12 und einem Endpunkt 21 der Bahn 18 oder der Bahn 12 aussendet, mit der optimierten Bewegungsbahn des Röntgendetektors 1.

Allerdings müssen aufgrund eines eventuell festgelegten Arbeitsbereichs (beispielsweise bei Verwendung der in der deutschen Anmeldung 10 2012 005 899.3 verwendeten Anordnung), der Startpunkt 15 und der Endpunkt 17 in der Höhe reduziert werden. Falls dies nötig ist, müssen auch entsprechend der maximale Öffnungswinkel α und der Startpunkt 22 und der Endpunkt 21 angepasst werden. In weiteren Ausführungsformen kann der maximale Öffnungswinkel, der erreicht werden soll, auch vorgegeben werden und davon ausgehend eine Anpassung erfolgen.

In Fig. 4 ist entsprechend der Abbildung 3 die Bahn 18 als durchgehende Bahn dargestellt. Eine Distanz der Röntgenquelle 2 zu dem Mittelpunkt 14 wurde für die optimierte polynomielle Bahn 18 derart angepasst, dass ein Abstand entlang des Zentralstrahls zwischen der Röntgenquelle 2 und dem Röntgendetektor 1, die sich im Verlauf der in Fig. 4 gezeigten Bahnen stets gegenüberliegen, immer konstant ist. Alternativ kann in weiteren Ausführungsbeispielen auch ein Abstand zwischen der Röntgenquelle 2 und dem Mittelpunkt 14 oder einer Oberfläche des Zielvolumens konstant sein. Es kann selbstverständlich auch eine variable Distanz der Röntgenquelle 2 zu dem Röntgendetektor 1 oder dem Mittelpunkt 14 bzw. der Oberfläche des Zielvolumens vorgesehen sein, um beispielsweise Kollisionen mit anderen Geräten zu vermeiden. Ebenso kann eine Ausrichtung des Röntgendetektors 1 orthogonal zu dem Zentralstrahl der Röntgenquelle 2 erfolgen, wie in Fig. 4 auch dargestellt. Es kann aber in weiteren Ausführungsbeispielen auch vorgesehen sein, dass die polynomielle Bewegungsbahn 13 des Röntgendetektors 1 möglichst nah an dem Patienten 7 bzw. dem OP-Tisch 6 verläuft. Hierdurch kann durch schräge Projektionsaufnahmen der Abstand der polynomiellen Bewegungsbahn zum OP-Tisch 6 und bzw. oder dem Patienten 7 weiter verringert werden. Die polynomielle Bewegungsbahnen 13 und 18 sind bezüglich eines Zentrums einer Bildebene des Röntgendetektors 1 definiert.

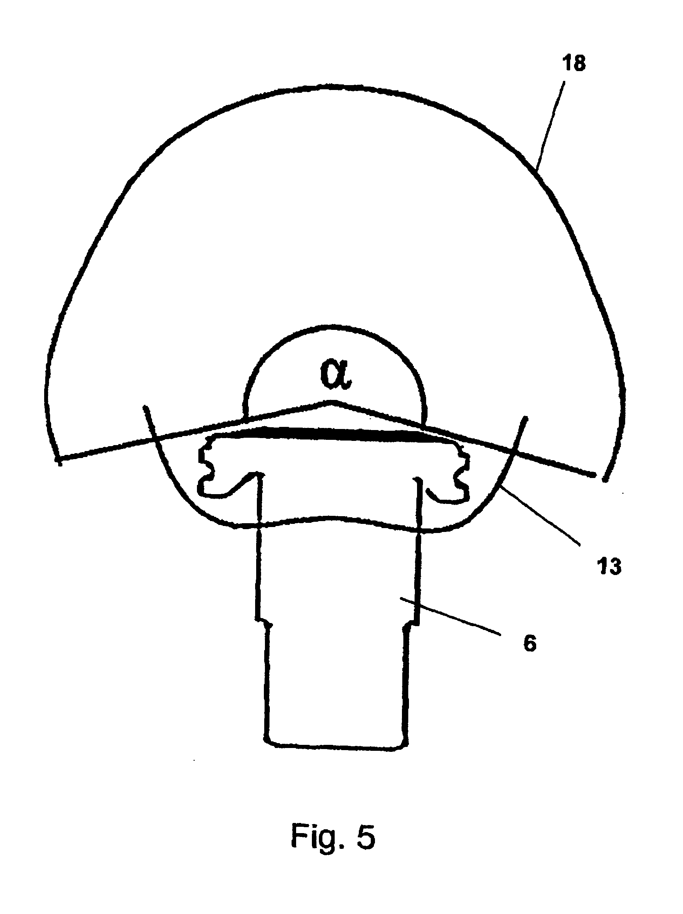

Fig. 5 stellt in einer Fig. 2 entsprechenden Ansicht die in Fig. 4 dargestellten, erhaltenen optimierten Bahnen 13 und 18 zusammen mit dem Öffnungswinkel α dar.

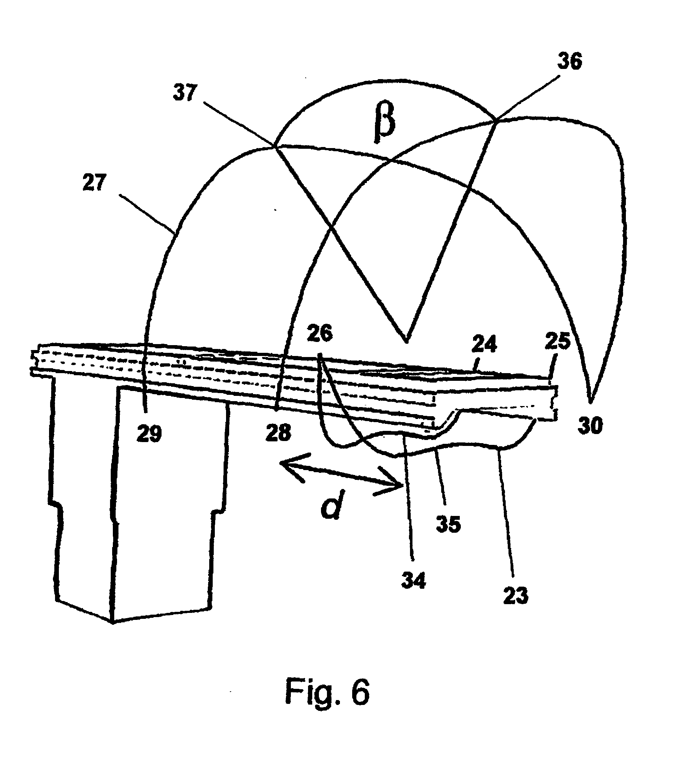

Wie in Fig. 6 in einer perspektivischen Ansicht gezeigt, kann zusätzlich zur seitlichen Auslenkung des Röntgendetektors 1 nach links und nach rechts vom OP-Tisch 6 auch durch die in der deutschen Patentanmeldung 10 2012 005 899.3 beschriebene Vorrichtung eine Verschiebung d in Längsrichtung des OP-Tischs 6 erfolgen. In Fig. 6 beträgt eine Länge dieser Verschiebung 300 mm. Typischerweise kann die Kinematik des Röntgendetektors 1 diesen um maximal 250 mm über eine Tischkante des OP-Tischs 6 hinaus verfahren, was auch den maximalen Öffnungswinkel α festlegt.

Alternativ oder, wie in Fig. 6, zusätzlich zu dieser Längsverschiebung vollführt der Röntgendetektor 1 eine Bewegung auf einer Bahn 23, bei der ein Startpunkt 24 und ein Endpunkt 25 zwar auf einer gleichen Seite des OP-Tischs 6, jedoch räumlich um den Betrag der Längsverschiebung zueinander versetzt liegen. Diesen Punkten 24, 25 gegenüberliegend befindet sich ein Umkehrpunkt 26, an dem der Röntgendetektor 1 auf der Bahn 23 einen Richtungswechsel der Bewegung vollführt. Demzufolge existieren auch zwei, räumlich zueinander versetzt liegende Mittelpositionen 34, 35 der Bewegung 23, in denen der Röntgendetektor 1 sich am nächsten zum Zielvolumen befindet. Der Röntgendetektor 1 bewegt sich also während des Bildaufnahmeverfahrens auf der polynomiellen Bahn 23 von einer Seite des Patienten 7 zur gegenüberliegenden Seite und wieder zurück. Simultan zu dieser Bewegung wird der Röntgendetektor 1 in Längsrichtung des OP-Tischs 6 verfahren. Generell können sowohl der Strahlendetektor 1 als auch die Strahlenquelle 2 um mindestens zwei Achsen bewegt werden, wobei diese Bewegungen sowohl Linearbewegungen als auch Rotationsbewegungen und beliebige Kombinationen sein können. Die beiden Achsen stehen hierbei typischerweise senkrecht aufeinander, jedoch sind in weiteren Ausführungsbeispielen auch beliebige geometrische Anordnungen der beiden Achsen zueinander denkbar.

Ebenso bewegt sich die Röntgenquelle 2 auf einer polynomiellen Bahn 27, die, da sich die Röntgenquelle 2 und der Röntgendetektor 1 einander gegenüberliegen während ihrer Bewegung, ebenfalls einen Startpunkt 28 und einen räumlich entlang einer Längsachse des OP-Tischs 6, also einer längsten Achse des OP-Tischs 6, räumlich versetzt zu diesem Startpunkt 28 liegenden Endpunkt 29. Diesen Punkten 28 und 29 gegenüber auf der anderen Seite des OP-Tischs 6 befindet sich ein Umkehrpunkt 30.

Auf einer Strecke zwischen dem Startpunkt 24 und dem ersten Mittelpunkt 34 bzw. zwischen dem zweiten Mittelpunkt 35 und einem Endpunkt 25 der Bahn 23 des Röntgendetektors 1 weist die Längsverschiebung ihre minimale bzw. maximale Auslenkung auf. Die Auslenkung kann in weiteren Ausführungsformen aber auch auf diesen Abschnitten variabel gestaltet werden, sodass beispielsweise die Auslenkung auf einem Abschnitt zwischen dem Startpunkt 24 und dem Umkehrpunkt 26 bzw. zwischen dem Umkehrpunkt 26 und dem Endpunkt 25 kontinuierlich erhöht wird.

In dem in Fig. 6 gezeigten Ausführungsbeispiel wird auf einer Strecke zwischen dem ersten Mittelpunkt und dem Umkehrpunkt 26 bzw. zwischen dem Umkehrpunkt 26 dem zweiten Mittelpunkt die Auslenkung des Röntgendetektors 1 kontinuierlich geändert. Hierfür wird die Auslenkung nach der mathematischen Funktion *d*=0,5**d*ₘₐₓ*cos(αᵢ/(0,5*α)) geändert, wobei αᵢ die aktuelle Winkelposition der Röntgenquelle 2 auf ihrer Bewegungsbahn 27 mit dem Öffnungswinkel α entspricht und *d*ₘₐₓ die maximale Auslenkung kennzeichnet. Es sind allerdings natürlich auch weitere Verläufe möglich. So kann die Bewegungsbahn 23 nicht nur auf den genannten Abschnitten, sondern auch auf einer Gesamtstrecke zwischen dem Startpunkt 24 und dem Umkehrpunkt 26 und bzw. oder auf einer Gesamtstrecke zwischen dem Umkehrpunkt 26 und dem Endpunkt 25 durch eine mathematische Funktion in kartesischen Koordinaten, Zylinder- oder Kugelkoordinaten definiert werden.

Die Röntgenquelle 2 wird in Abhängigkeit von der Längsverschiebung d des Röntgendetektors 1 in entgegengesetzter Richtung um den Öffnungswinkel β in dem in Fig. 6 gezeigten Ausführungsbeispiel geneigt, sodass der Zentralstrahl stets auf den Röntgendetektor 1 gerichtet ist. Die Neigung β wird entsprechend der Detektorauslenkung derart berechnet, dass eine bestrahlte Sensorfläche des Röntgendetektors 1 bei einem festen Kegelstrahlöffnungswinkel γ maximal wird. Die Neigung entspricht hierbei einem maximalen Winkel zwischen einer ersten Mittelposition 36 und einer zweiten Mittelposition 37 der Bahn 27. Die Mittelpositionen 36, 37 der Bahn 27 liegen hierbei den Mittelpositionen 34, 35 der Bahn 23 gegenüber. Alternativ oder zusätzlich kann für die Berechnung der Neigung β eine vollständige Abbildung einer vorgegebenen Volumengröße auf der Sensorfläche Bedingung sein. In dem in Fig. 6 gezeigten Ausführungsbeispiel ist somit in den Mittelstellungen 34, 35 der Bahn 23 die Neigung β der Röntgenquelle 2 maximal. Durch die Neigung β kann auch die Distanz wie zuvor bereits beschrieben angepasst werden, d. h. beispielsweise kann ein konstanter Abstand zwischen der Röntgenquelle 2 und dem Röntgendetektor 1 bei jeder Position, in der eine Projektionsaufnahme gemacht wird, eingestellt werden.

Das in Bezug auf die Bahn 23 des Röntgendetektors 1 Gesagte gilt natürlich in gleicher Weise für die Bahn 27 der Röntgenquelle 2.

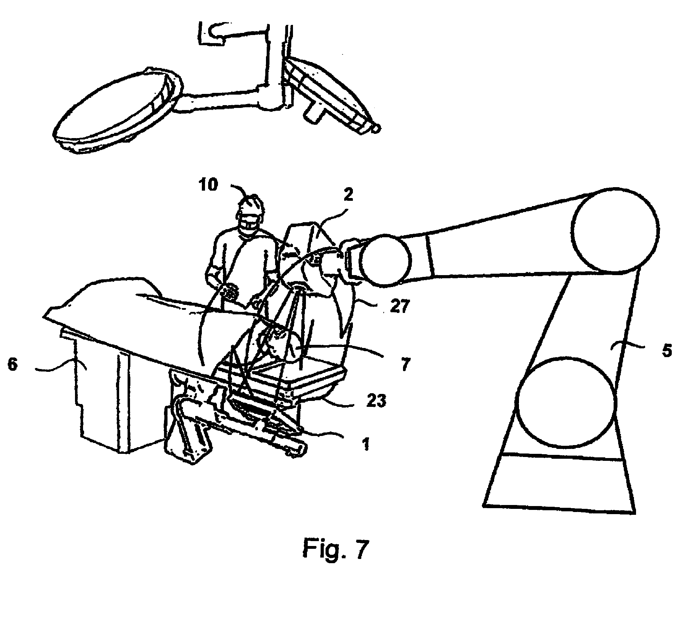

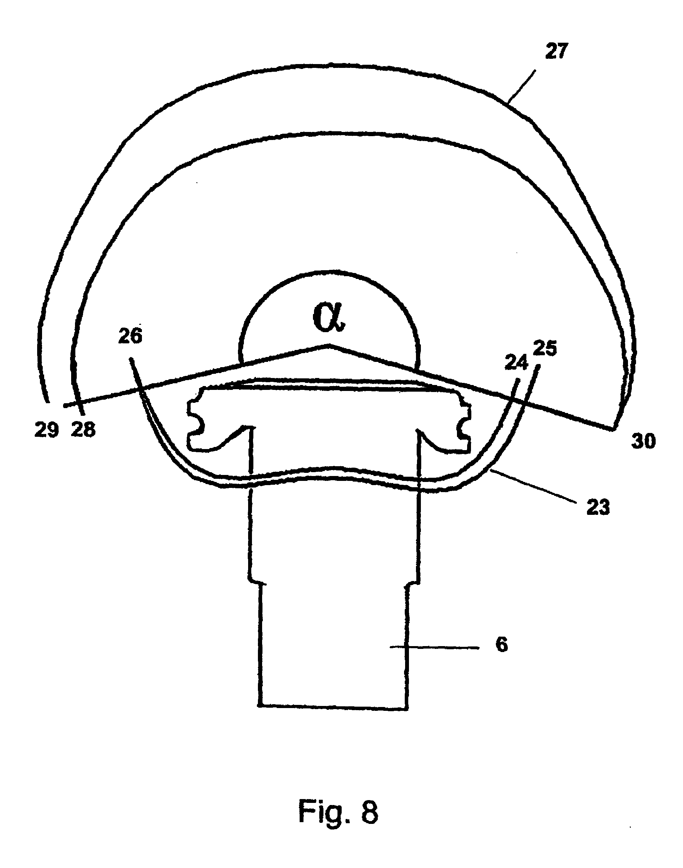

Fig. 7 stellt in einer Fig. 1 entsprechenden perspektivischen Ansicht die Bewegungsbahnen 23 und 27 zusammen mit dem Roboterarm 5, dem Patienten 7 und dem Röntgendetektor 1 dar. Ebenso sind in Fig. 8 in einer Fig. 2 entsprechenden Darstellungen die Bewegungsbahnen 23 und 27 wiedergegeben.

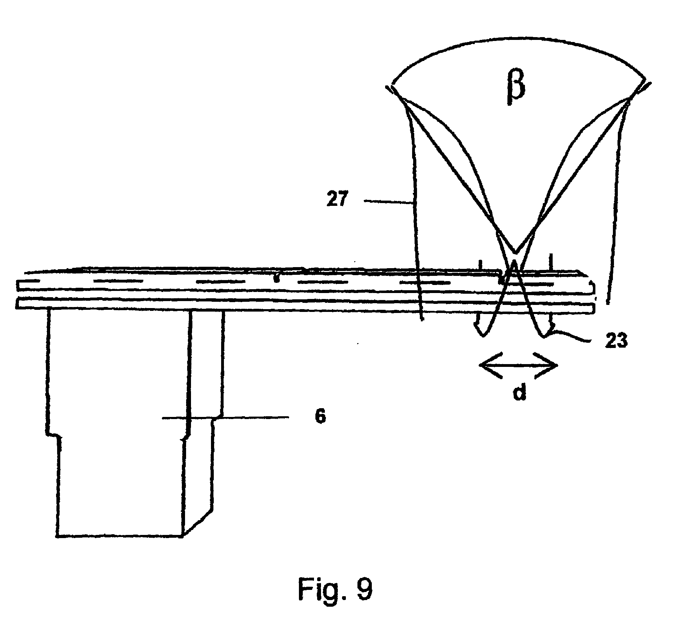

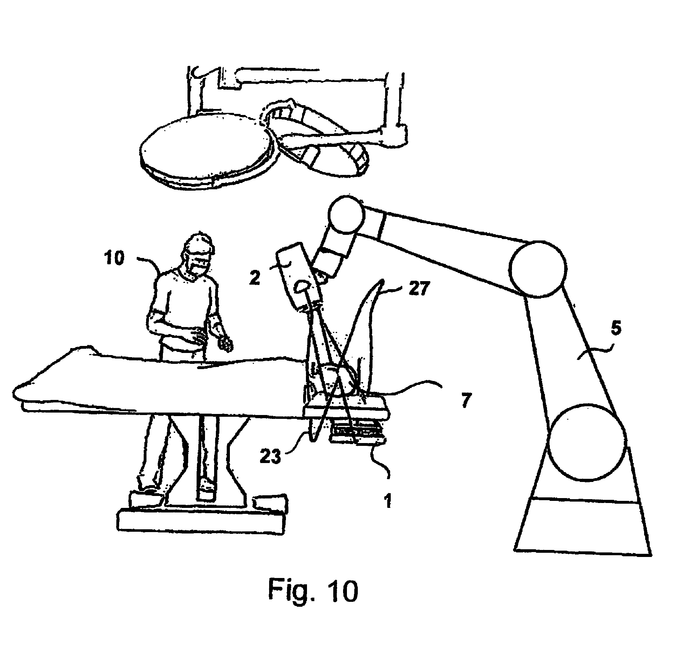

In Fig. 9 ist schließlich eine Seitenansicht der Fig. 8 gezeigt, aus der deutlich die Verschiebung in Längsrichtung der Bahn 23 des Röntgendetektors 1 sowie die Neigung β der Röntgenquelle 2 samt deren Bahn 27 hervorgeht. Fig. 10 zeigt in einer seitlichen Ansicht der Vorrichtung mit den in den Fign. 8 und 9 gezeigten Bewegungsbahnen, wie mittels des Roboterarms 5 die Röntgenquelle 2 auf ihrer Bahn bewegt und geneigt werden kann. Ferner ist auch die Bahn 23 des Röntgendetektors 1 eingezeichnet. Die Bahnen 23 und 27 sind offen, weisen also jeweils einen nicht zusammenfallenden Anfangs- und Endpunkt auf und umschließen den Patienten 7 nicht vollständig.

In weiteren Ausführungsbeispielen kann eine der Bewegungsbahnen der Röntgenquelle 2 und des Röntgendetektors 1 oder auch beide Bewegungsbahnen mehrfach aneinandergereiht werden. In diesem Fall werden mehrere Umkehrpunkte zwischen den jeweiligen Anfangs- und Endpunkten verwendet.

Alternativ oder zusätzlich kann auch die gesamte Bildaufnahmebahn bzw. können beide Bildaufnahmebahnen um das Zielvolumen bzw. den Patienten 7 und den OP-Tisch 6 rotiert werden.

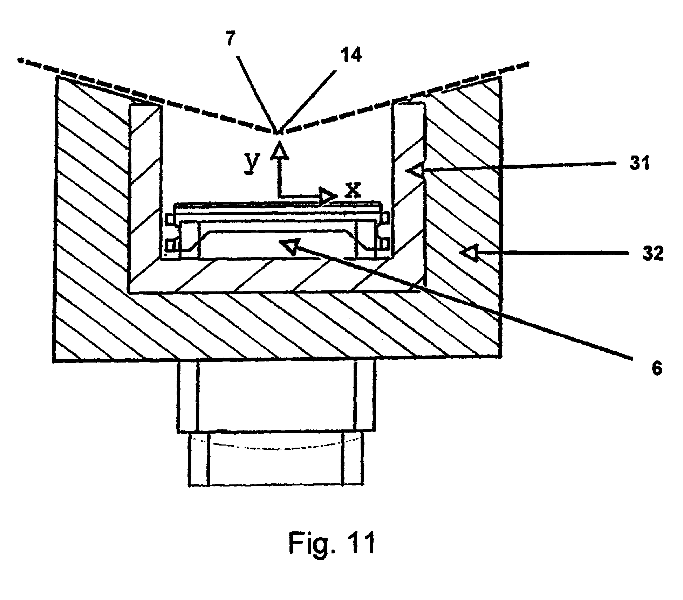

Fig. 11 zeigt eine Schnittansicht des OP-Tischs 6, bei dem um den Patienten 7 ein kastenförmiger Sicherheitsbereich 31 gezogen ist, der bei der Ermittlung des polynomiellen Verfahrens berücksichtigt wird. Als Bedingung wird verwendet, dass ein Gehäuse der Röntgenquelle 2 und bzw. oder des Röntgendetektors 1 stets mindestens 80 mm vom OP-Tisch 6 und vom Patienten 7 entfernt sein muss. Zusätzlich ist ein Arbeitsbereich 32 als weitere Bedingung für die polynomielle Bewegungsbahn des Röntgendetektors 1 vorgegeben. Dieser Arbeitsbereich 32 berücksichtigt einen maximalen Abstand zum Patienten 7 und bzw. oder einen Arbeitsbereich einer Kinematik zum Verfahren des Röntgendetektors 1. Der Arbeitsbereich 32 beschreibt hierbei die Positionen seitlich und unterhalb des OP-Tischs 6, in die der Röntgendetektor 1 verfahrbar ist.

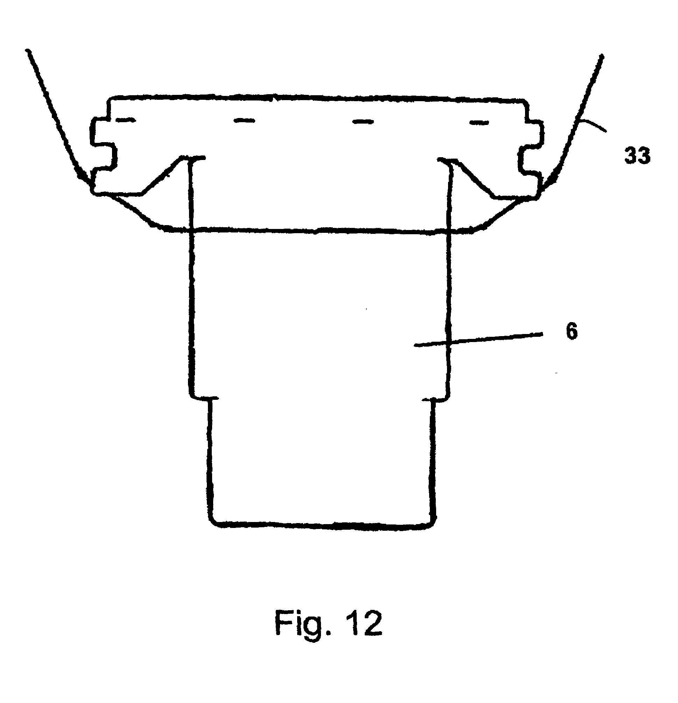

Fig. 12 stellt schließlich in einer Fig. 2 entsprechenden Darstellung eine weitere Möglichkeit einer Ausgangsbahn 33 für die Optimierung der Bahn des Röntgendetektors 1 dar. Statt einer kreisbogenförmigen Bahn wird eine aus einzelnen linearen Abschnitten bestehende Bahn 33 verwendet. Ein mittlerer Abschnitt unterhalb des OP-Tischs 6 verläuft parallel zu einer Oberfläche des OP-Tischs 6. Anschließend an diesen mittleren Teil werden abgewinkelte, wiederum linear verlaufende Teilstücke zum Vervollständigen der Bahn verwendet.

Lediglich in den Ausführungsbeispielen offenbarte Merkmale der verschiedenen Ausführungsformen können miteinander kombiniert und einzeln beansprucht werden.

### Bezugszeichenliste:

- 1: Röntgendetektor
- 2: Röntgenquelle
- 3: Steuereinheit
- 4: Auswerteeinheit
- 5: Roboterarm
- 6: OP-Tisch
- 7: Patient
- 8: Kabel
- 9: Kabel
- 10: Benutzer
- 11: Bildschirm
- 12: Bahn
- 13: polynomielle Bahn
- 14: Mittelpunkt
- 15: Startposition
- 16: Mittelposition
- 17: Endposition
- 18: polynomielle Bahn
- 19: Zentralstrahl
- 20: Zentralstrahl
- 21: Endpunkt
- 22: Startpunkt
- 23: polynomielle Bahn
- 24: Startpunkt
- 25: Endpunkt
- 26: Umkehrpunkt
- 27: polynomielle Bahn
- 28: Startpunkt
- 29: Endpunkt
- 30: Umkehrpunkt
- 31: Sicherheitsbereich
- 32: Arbeitsbereich
- 33: Bahn
- 34: Mittelposition
- 35: Mittelposition
- 36: Mittelposition
- 37: Mittelposition
- 38: Kabel

## Patentansprüche

1. Verfahren zum Aufnehmen von Projektionsbildern eines abzubildenden Objekts (7) durch eine Abbildungsvorrichtung, die eine Strahlenquelle (2), einen Strahlendetektor (1) und eine Steuereinheit (3) aufweist, wobei die Strahlenquelle (2) durch die Steuereinheit (3) auf einer Bahn (12, 18, 27) in mehrere Positionen verfahren wird, in denen jeweils eine von der Strahlenquelle (2) kegelstrahlförmig um einen Zentralstrahl (19, 20) ausgehende Strahlung ausgesandt wird, und mit dem Verfahren der Strahlenquelle (2) der Strahlendetektor (1) durch die Steuereinheit (3) in mehrere Positionen verfahren wird, in denen jeweils die von der Strahlenquelle (2) ausgehende und das abzubildende Objekt (7) durchdringende Strahlung zum Aufnehmen eines Projektionsbilds aufgefangen wird,
**dadurch gekennzeichnet, dass**
die Strahlenquelle (2) und/oder der Strahlendetektor (1) jeweils auf einer durch ein Polynom n-ten Grades beschriebenen und von der Steuereinheit (3) durch eine Optimierung einer vorgegebenen Bahn (12, 33) mit entlang des Zentralstrahls (19, 20) bestimmtem, konstantem Abstand zwischen der Strahlenquelle (2) und dem Strahlendetektor (1) und/oder mit entlang des Zentralstrahls (19, 20) bestimmtem, konstantem Abstand zwischen der Strahlenquelle (2) und dem abzubildenden Objekt (7) berechneten Bahn (13, 18, 23, 27) um das abzubildende Objekt (7) verfahren werden, wobei das Polynom jeweils derart gewählt ist, dass ein Sicherheitsabstand zu dem abzubildenden Objekt (7) eingehalten wird und gleichzeitig ein Abstand zwischen dem Strahlendetektor (1) und dem abzubildenden Objekt (7) minimiert wird.

2. Verfahren nach Anspruch 1, **dadurch gekennzeichnet, dass** das Polynom durch mindestens zwei Stützstellen definiert ist und vorzugsweise durch eine Optimierung unter Berücksichtigung der Stützstellen und/oder durch eine Optimierung einer vorgegebenen kreisförmigen Bahn (12) bestimmt wird.

3. Verfahren nach Anspruch 1 oder Anspruch 2, **dadurch gekennzeichnet, dass** eine maximale Höhe der Bahn (13, 23) des Strahlendetektors (1) durch einen ausgehend von einem Mittelpunkt (14) des abzubildenden Objekts (7) bestimmten maximalen Öffnungswinkel (α) der Bahn (12, 18, 27) der Strahlenquelle (2) bestimmt wird, wobei vorzugsweise ein Startpunkt (24) und ein Endpunkt (25) der Bahn (23) des Strahlendetektors (1) einem Startpunkt (28) und einem Endpunkt (29) der Bahn (27) der Strahlenquelle (2) entlang des durch den Mittelpunkt (14) des abzubildenden Objekts verlaufenden Zentralstrahls (19, 20) gegenüberliegen.

4. Verfahren nach einem der vorhergehenden Ansprüche, **dadurch gekennzeichnet, dass** die Bahn der Bewegung der Strahlenquelle (2) und/oder des Strahlendetektors (1) offen ist und/oder mindestens einen Umkehrpunkt (26, 30) aufweist, in dem ein Richtungswechsel stattfindet, wobei vorzugsweise ein erster Bahnabschnitt bis zu dem Umkehrpunkt (26, 30) räumlich verschieden von einem zweiten Bahnabschnitt ab dem Umkehrpunkt (26, 30) ist.

5. Verfahren nach einem der vorhergehenden Ansprüche, **dadurch gekennzeichnet, dass** der Strahlendetektor (1) und/oder die Strahlenquelle (2) entlang von und/oder um mindestens zwei, vorzugsweise senkrecht aufeinander stehenden Achsen bewegt wird.

6. Verfahren nach einem der vorhergehenden Ansprüche, **dadurch gekennzeichnet, dass** eine Neigung (β) der Strahlenquelle (2) derart eingestellt wird, dass eine bestrahlte Fläche des Strahlendetektors (1) bei einem festen Winkel des Kegelstrahls maximiert ist.

7. Verfahren nach einem der vorhergehenden Ansprüche, **dadurch gekennzeichnet, dass** zumindest eine der Bewegungen der Strahlenquelle (2) und des Strahlendetektors (1) automatisiert durch die Steuereinheit (3) durchgeführt werden.

8. Verfahren nach einem der vorhergehenden Ansprüche, **dadurch gekennzeichnet, dass** eine Auswerteeinheit (4) eine dreidimensionale Rekonstruktion des abzubildenden Objekts (7) aus den Projektionsbildern berechnet.

9. Vorrichtung zum Aufnehmen von Projektionsbildern, umfassend einen Strahlendetektor (1), eine Strahlenquelle (2) und eine Steuereinheit (3), wobei die Steuereinheit (3) eingerichtet ist, den Strahlendetektor (1) und die Strahlenquelle (2) jeweils in mehrere Positionen zu verfahren und in jeder der Positionen ein Projektionsbild aufzunehmen, die Strahlenquelle (2) eingerichtet ist, eine Strahlung kegelstrahlförmig um einen Zentralstrahl (19, 20) auszusenden, und der Strahlendetektor (1) eingerichtet ist, die ausgesandte und das abzubildende Objekt (7) durchdringende Strahlung aufzufangen,
**dadurch gekennzeichnet, dass**
die Steuereinheit (3) dazu ausgebildet ist, die Strahlenquelle (2) und den Strahlendetektor (1) jeweils auf einer durch ein Polynom n-ten Grades beschriebenen und von der Steuereinheit durch eine Optimierung einer vorgegebenen Bahn (12, 33) mit entlang des Zentralstrahls (19, 20) bestimmtem, konstantem Abstand zwischen der Strahlenquelle (2) und-dem Strahlendetektor (1) und/oder mit entlang des Zentralstrahls (19, 20) bestimmtem, konstantem Abstand zwischen der Strahlenquelle (2) und dem abzubildenden Objekt (7) berechneten Bahn (13, 18, 23, 27) um das abzubildende Objekt (7) zu verfahren, wobei das Polynom jeweils derart gewählt ist, einen Sicherheitsabstand zu dem abzubildenden Objekt einzuhalten und gleichzeitig einen Abstand zwischen dem Strahlendetektor (1) und dem abzubildenden Objekt (7) zu minimieren.

10. Computerprogrammprodukt, enthaltend eine auf einem maschinenlesbaren Träger gespeicherte Befehlsfolge zum Durchführen des Verfahrens nach einem der Ansprüche 1-8 und/oder zum Ansteuern der Vorrichtung nach Anspruch 9, wenn das Computerprogrammprodukt auf einer Recheneinheit abläuft.

## Claims

1. Method for capturing projection images of an object (7) to be imaged by an imaging device which comprises a radiation source (2), a radiation detector (1) and a control unit (3), wherein the radiation source (2) is moved by the control unit (3) on a path (12, 18, 27) into a plurality of positions, in each of which radiation is emitted by the radiation source (2) in a cone beam shape around a central beam (19, 20), and with the movement of the radiation source (2), the radiation detector (1) is moved by the control unit (3) into a plurality of positions, in each of which the radiation emitted by the radiation source (2) and penetrating the object (7) to be imaged is intercepted to capture a projection image,
**characterised in that**
the radiation source (2) and/or the radiation detector (1) are moved in each case on a calculated path (13, 18, 23, 27) around the object (7) to be imaged, which path is described by a polynomial of nth degree and determined by the control unit (3) through an optimisation of a predetermined path (12, 33) at a constant distance, determined along the central beam (19, 20), between the radiation source (2) and the radiation detector (1) and/or at a constant distance, determined along the central beam (19, 20), between the radiation source (2) and the object (7) to be imaged, wherein the polynomial is selected in each case in such a manner that a safety clearance with respect to the object (7) to be imaged is maintained and, at the same time, a distance between the radiation detector (1) and the object (7) to be imaged is minimised.

2. Method according to Claim 1, **characterised in that** the polynomial is defined by at least two nodes and is preferably determined by an optimisation taking into account the nodes and/or by an optimisation of a predetermined circular path (12).

3. Method according to Claim 1 or Claim 2, **characterised in that** a maximum height of the path (13, 23) of the radiation detector (1) is determined by a maximum spread angle (α) of the path (12, 18, 27) of the radiation source (2), which angle is determined starting from a centre point (14) of the object (7) to be imaged, wherein preferably a starting point (24) and an end point (25) of the path (23) of the radiation detector (1) lie opposite a starting point (28) and an end point (29) of the path (27) of the radiation source (2) along the central beam (19, 20) running through the centre point (14) of the object to be imaged.

4. Method according to one of the preceding claims, **characterised in that** the path of the movement of the radiation source (2) and/or of the radiation detector (1) is open and/or has at least one reversal point (26, 30), in which a change of direction takes place, wherein preferably a first path section up to the reversal point (26, 30) is spatially different from a second path section from the reversal point (26, 30).

5. Method according to one of the preceding claims, **characterised in that** the radiation detector (1) and/or the radiation source (2) is moved along and/or around at least two, preferably mutually perpendicular axes.

6. Method according to one of the preceding claims, **characterised in that** an inclination (β) of the radiation source (2) is set such that an irradiated area of the radiation detector (1) is maximised at a fixed angle of the cone beam.

7. Method according to one of the preceding claims, **characterised in that** at least one of the movements of the radiation source (2) and of the radiation detector (1) are carried out in an automated manner by the control unit (3).

8. Method according to one of the preceding claims, **characterised in that** an evaluation unit (4) calculates a three-dimensional reconstruction of the object (7) to be imaged, from the projection images.

9. Device for capturing projection images, comprising a radiation detector (1), a radiation source (2) and a control unit (3), wherein the control unit (3) is adapted to move the radiation detector (1) and the radiation source (2) in each case into a plurality of positions and to capture a projection image in each of the positions, the radiation source (2) is adapted to emit radiation in a cone beam shape around a central beam (19, 20), and the radiation detector (1) is adapted to intercept the radiation emitted and penetrating the object (7) to be imaged,
**characterised in that**
the control unit (3) is configured to move the radiation source (2) and the radiation detector (1) in each case on a calculated path (13, 18, 23, 27) around the object (7) to be imaged, which path is described by a polynomial of nth degree and determined by the control unit through an optimisation of a predetermined path (12, 33) at a constant distance, determined along the central beam (19, 20), between the radiation source (2) and the radiation detector (1) and/or at a constant distance, determined along the central beam (19, 20), between the radiation source (2) and the object (7) to be imaged, wherein the polynomial is selected in each case in such a manner as to maintain a safety clearance with respect to the object to be imaged and, at the same time, minimise a distance between the radiation detector (1) and the object (7) to be imaged.

10. Computer program product, comprising an instruction sequence, stored on a machine-readable medium, for carrying out the method according to one of Claims 1-8 and/or for controlling the device according to Claim 9 when the computer program product is running on a computing unit.

## Revendications

1. Procédé de prise d'images de projection d'un objet (7) à imager par un dispositif d'imagerie, qui comprend une source de rayonnement (2), un détecteur de rayonnement (1) et une unité de commande (3), la source de rayonnement (2) étant déplacée par l'unité de commande (3) sur une trajectoire (12, 18, 27) vers plusieurs positions, dans lesquelles un rayonnement sortant sous la forme d'un faisceau conique de la source de rayonnement (2) est émis autour d'un faisceau central (19, 20) et, grâce au déplacement de la source de rayonnement (2), le détecteur de rayonnement (1) est déplacé par l'unité de commande (3) vers plusieurs positions, dans lesquelles le rayonnement sortant de la source de rayonnement (2) et traversant l'objet à imager (7) est capturé pour la prise d'une image de projection,
**caractérisé en ce que**
la source de rayonnement (2) et/ou le détecteur de rayonnement (1) sont déplacés sur une trajectoire (13, 18, 23, 27) calculée autour de l'objet à imager (7) décrite par un polynôme du n-ième degré par l'unité de commande (3), grâce à une optimisation d'une trajectoire prédéterminée (12, 33), avec une distance constante déterminée le long du faisceau central (19, 20), entre la source de rayonnement (2) et le détecteur de rayonnement (1) et/ou avec une distance constante déterminée le long du faisceau central (19, 20) entre la source de rayonnement (2) et l'objet à imager (7), le polynôme étant choisi de telle sorte qu'une distance de sécurité par rapport à l'objet à imager (7) soit respectée et qu'une distance entre le détecteur de rayonnement (1) et l'objet à imager (7) soit en même temps minimisé.

2. Procédé selon la revendication 1, **caractérisé en ce que** le polynôme est défini par au moins deux points de base et est déterminé de préférence par une optimisation en tenant compte des points de base et/ou par une optimisation d'une trajectoire circulaire (12) prédéterminée.

3. Procédé selon la revendication 1 ou la revendication 2, **caractérisé en ce qu'**une hauteur maximale de la trajectoire (13, 23) du détecteur de rayonnement (1) est déterminée par un angle d'ouverture (α) maximal, à partir d'un centre (14) de l'objet à imager (7), de la trajectoire (12, 18, 27) de la source de rayonnement (2), de préférence un point de départ (24) et un point d'arrivée (25) de la trajectoire (23) du détecteur de rayonnement (1) faisant face à un point de départ (28) et à un point d'arrivée (29) de la trajectoire (27) de la source de rayonnement (2) le long du faisceau central (19, 20) qui traverse le centre (14) de l'objet à imager.

4. Procédé selon l'une des revendications précédentes, **caractérisé en ce que** la trajectoire du mouvement de la source de rayonnement (2) et/ou du détecteur de rayonnement (1) est ouverte et/ou présente au moins un point d'inversion (26, 30) au niveau duquel un changement de direction a lieu, de préférence une première portion de trajectoire est, jusqu'au point d'inversion (26, 30), spatialement distincte d'une deuxième portion de trajectoire à partir du point d'inversion (26, 30).

5. Procédé selon l'une des revendications précédentes, **caractérisé en ce que** le détecteur de rayonnement (1) et/ou la source de rayonnement (2) sont déplacés le long et/ou autour d'au moins, de préférence, deux axes perpendiculaires entre eux.

6. Procédé selon l'une des revendications précédentes, **caractérisé en ce qu'**une inclinaison (β) de la source de rayonnement (2) est réglée de façon à ce qu'une surface irradiée du détecteur de rayonnement (1) est maximisée pour un angle fixe du faisceau conique.

7. Procédé selon l'une des revendications précédentes, **caractérisé en ce qu'**au moins un des mouvements de la source de rayonnement (2) et du détecteur de rayonnement (1) est effectué de manière automatique par l'unité de commande (3).

8. Procédé selon l'une des revendications précédentes, **caractérisé en ce qu'**une unité d'analyse calcule une reconstruction tridimensionnelle de l'objet à imager (7) à partir des images de projection.

9. Procédé de prise d'images de projection, comprenant un détecteur de rayonnement (1), une source de rayonnement (2) et une unité de commande (3), l'unité de commande (3) étant conçue pour déplacer le détecteur de rayonnement (1) et la source de rayonnement (2) vers plusieurs positions et pour prendre une image de projection au niveau de chacune des positions, la source de rayonnement (2) étant conçue pour émettre un rayonnement sous la forme d'un faisceau conique autour d'un faisceau central (19, 20) et le détecteur de rayonnement (1) étant conçu pour capturer le rayonnement émis et traversant l'objet à imager (7),
**caractérisé en ce que**
l'unité de commande (3) est conçue pour déplacer la source de rayonnement (2) et le détecteur de rayonnement (1) sur une trajectoire (13, 18, 23, 27) calculée autour de l'objet à imager (7), décrite par un polynôme du n-ième degré, grâce à une optimisation d'une trajectoire prédéterminée (12, 33), avec une distance déterminée constante, le long du faisceau central (19, 20), entre la source de rayonnement (2) et le détecteur de rayonnement (1) et/ou avec une distance déterminée constante, le long du faisceau central (19, 20), entre la source de rayonnement (2) et l'objet à imager (7), le polynôme étant choisi de façon à ce qu'une distance de sécurité par rapport à l'objet à imager soit respectée et à ce qu'une distance entre le détecteur de rayonnement (1) et l'objet à imager (7) soit en même temps minimisée.

10. Produit logiciel contenant une suite d'instructions enregistrées sur un support lisible par une machine, pour la réalisation du procédé selon l'une des revendications 1 à 8 et/ou pour la commande du dispositif selon la revendication 9 lorsque le produit logiciel est exécuté sur un ordinateur.
